(19) Europäisches Patentamt
European Patent Office
Office européen des brevets

(11) EP 1 787 680 A2

(12) **DEMANDE DE BREVET EUROPEEN**

(43) Date de publication:
**23.05.2007 Bulletin 2007/21**

(21) Numéro de dépôt: **06291471.8**

(22) Date de dépôt: **20.09.2006**

(51) Int Cl.:
***A61Q 1/10*** *(2006.01)* ***A61Q 5/06*** *(2006.01)*
***A61K 8/40*** *(2006.01)* ***A61K 8/81*** *(2006.01)*
***A61K 8/894*** *(2006.01)* ***A61K 8/897*** *(2006.01)*

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **07.10.2005 FR 0510276**

(71) Demandeur: **L'Oreal**
**75008 Paris (FR)**

(72) Inventeurs:
- **Livoreil, Aude**
**75006 Paris (FR)**
- **Brun, Gaëlle**
**75015 Paris (FR)**

(74) Mandataire: **Casalonga, Axel**
**Bureau Casalonga & Josse**
**Bayerstrasse 71/73**
**80335 München (DE)**

(54) **Composition cosmétique à base de monomères électrophiles et d'une dispersion de particules de polymère stabilisées en surface**

(57) La présente demande a pour objet une composition cosmétique, notamment capillaire, caractérisée en ce qu'elle comprend au moins un monomère électrophile, une phase grasse liquide, et des particules de polymère dispersées dans la phase grasse liquide et stabilisées en surface par un agent stabilisant.

EP 1 787 680 A2

## Description

La présente invention est relative à des compositions cosmétiques à base de monomères électrophiles et d'une dispersion de particules de polymère stabilisées en surface, ainsi qu'à l'utilisation de ces compositions pour le traitement des matières kératiniques, et notamment des fibres kératiniques telles que les cheveux.

Par "matières kératiniques", on entend de préférence les fibres kératiniques, et encore de préférence les cheveux.

Il existe de nombreux produits de coiffage permettant d'apporter du volume aux cheveux. Un inconvénient lié à ces produits, le plus souvent à base de polymères filmogènes, réside dans le fait que l'effet cosmétique disparaît dès le premier shampooing.

On connaît par ailleurs des traitements permanents des fibres kératiniques. Ces traitements utilisent un agent réducteur et un agent oxydant, et nécessitent une mise sous tension mécanique des fibres à l'aide d'un matériel d'enroulage, afin de conférer une mise en forme. Ces procédés, qui permettent effectivement d'augmenter le volume de la chevelure, présentent toutefois l'inconvénient de modifier le niveau de frisure de la chevelure et de dégrader le toucher de la fibre.

Il apparaît ainsi nécessaire de développer des compositions permettant d'accroître le volume de la chevelure sans modifier la forme ou le toucher des cheveux, le tout étant rémanent aux shampooings.

La demanderesse vient de découvrir de manière surprenante qu'il était possible d'atteindre les objectifs mentionnés ci-dessus, en mettant en oeuvre des compositions à base de monomères électrophiles et d'une dispersion de particules de polymère stabilisées en surface par un agent stabilisant dans un milieu non aqueux.

En particulier, la demanderesse a constaté que ces compositions conféraient du volume à la coiffure, sans altération du toucher des cheveux, ni de leur forme, sans dégradation des cheveux et sans adhésion des cheveux entre eux. En outre, ces propriétés cosmétiques sont rémanentes aux shampooings.

L'invention a donc pour objet une composition cosmétique, notamment capillaire, caractérisée en ce qu'elle comprend au moins un monomère électrophile, une phase grasse liquide et des particules de polymère dispersées dans la phase grasse liquide et stabilisées en surface par un agent stabilisant.

L'invention a également pour objet l'utilisation de cette composition pour le traitement cosmétique des matières kératiniques, et notamment des fibres kératiniques telles que les cheveux, ainsi que l'utilisation de cette composition pour conférer du volume aux fibres kératiniques.

L'invention a également pour objet un procédé de traitement cosmétique des matières kératiniques, et notamment des fibres kératiniques telles que les cheveux, comprenant l'application sur les matières kératiniques d'une composition selon l'invention.

Elle a aussi pour objet un kit comprenant une première composition contenant au moins un monomère électrophile (présent à des teneurs pouvant être comprise entre 0,5 et 50% du poids de la première composition) et éventuellement au moins un inhibiteur de polymérisation anionique et/ou radicalaire (présent à des teneurs pouvant être comprise entre 10 ppm et 5% du poids de la première composition), ainsi qu'une deuxième composition comprenant une phase grasse liquide et des particules de polymère dispersées dans la phase grasse liquide et stabilisées en surface par un agent stabilisant (présentes à des teneurs pouvant être comprise entre 0,001 et 5% du poids de la deuxième composition).

D'autres objets de l'invention apparaîtront à la lecture de la description et des exemples qui suivent.

Par monomère électrophile, on entend un monomère capable de polymériser par polymérisation anionique en présence d'un agent nucléophile.

Par polymérisation anionique, on entend le mécanisme défini dans l'ouvrage "Advanced Organic Chemistry", Third Edition de Jerry March, pages 151 à 161. Dans le cadre d'une polymérisation anionique, l'agent nucléophile (tel que par exemple, les ions hydroxyles OH- contenus dans l'eau) initie la polymérisation par la formation d'un carbanion au contact du monomère électrophile.

On entend par "carbanion ", les espèces chimiques définies dans " Advanced Organic Chemistry, Third Edition ", de Jerry March, page 141.

Selon un mode particulier de réalisation, le monomère électrophile répond à la formule (A) :

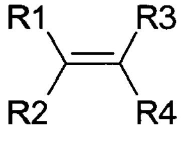 (A)

dans laquelle :

$R_1$ et $R_2$ désignent chacun, indépendamment l'un de l'autre, un groupe peu ou non électro-attracteur (peu ou non inductif-attracteur) tel que :

- un atome d'hydrogène,
- un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR,
- OH, et les atomes d'halogène,
- un résidu polyorganosiloxane modifié ou non,
- un groupement polyoxyalkylène,

$R_3$ et $R_4$ désignent chacun, indépendamment l'un de l'autre, un groupe électro-attracteur (ou inductif-attacteur) choisi de préférence parmi les groupements $-N(R)_3^+$, $-S(R)_2^+$, $-SH_2^+$, $-NH_3^+$, $-NO_2$, $-SO_2R$, -C≡N, -COOH, -COOR, -COSR, $-CONH_2$, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en $C_1$-$C_4$, les groupements aryle tels que phényle, ou les groupements aryloxy tels que phénoxyloxy,

R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un radical alkyle en $C_1$-$C_{10}$.
Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).
Par groupement peu ou non électro-attracteur, on entend tout groupement dont l'électronégativité est inférieure ou égale à celle du carbone.
Les groupements alcényle ou alcynyle ont de préférence 2 à 20 atomes de carbone, mieux encore de 2 à 10 atomes de carbone.
Comme groupe hydrocarboné saturé ou non, linéaire, ramifié ou cyclique, comportant de préférence de 1 à 20 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.
Comme groupe hydrocarboné substitué, on peut citer par exemple les groupes hydroxyalkyle ou polyhalogénoalkyle.
A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.
Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.
Parmi les groupements polyoxyalkylène, on peut notamment citer les groupements polyoxyéthylène et les groupements polyoxypropylène ayant de préférence 1 à 200 motifs oxyalkylénés.
Parmi les groupements mono- ou polyfluoroalkyle, on peut notamment citer des groupements tels que -(CH2) n-(CF2)m-CF3 ou -(CH2)n-(CF2)m-CHF2 avec n=1 à 20 et m= 1 à 20.
Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.
A titre d'exemples de groupement à fonction colorante, on peut notamment citer les groupements azoiques, quinoniques, méthiniques, cyanométhiniques et triarylméthane.
A titre d'exemples de groupement à fonction antioxydante, on peut notamment citer les groupements de type butylhydroxyanisole (BHA), butylhydroxytoluène (BHT) ou vitamine E.
A titre d'exemples de groupement à fonction filtre UV, on peut notamment citer les groupements de types benzophénones, cinnamates, benzoates, benzylidène-camphres et dibenzoylméthanes.
A titre d'exemples de groupement à fonction conditionnante, on peut notamment citer les groupements cationiques et de type esters gras.
Parmi les monomères électrophiles répondant à la formule (A), on peut citer par exemple :

- les dérivés benzylidene malononitrile de formule

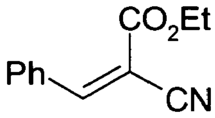

- le 2-(4-chloro-benzylidene)-malononitrile de formule

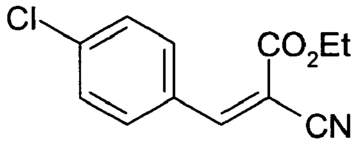

- le 2-cyano-3-phényl acrylate d'éthyle,

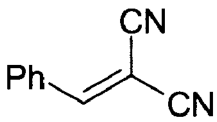

- le 2-cyano-3-(4-chloro-phényl) acrylate d'éthyle

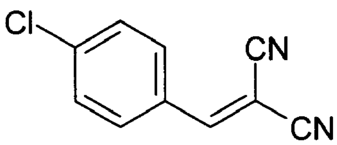

décrits dans Sayyah, J. Polymer Research, 2000, p 97,

- les dérivés de méthylidenemalonates comme :

  le 2-méthylene-malonate de diéthyle décrits dans les références suivantes par Hopff, Makromoleculare Chemie, 1961, p95, De Keyser, J. Pharm. Sci, 1991, p67 et Klemarczyk, Polymer, 1998, p173

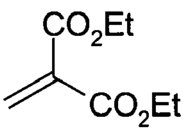

  le 2-éthoxycarbonylméthyleneoxycarbonyl acrylate d'éthyle décrits dans les références suivantes par Breton, Biomaterials, 1998, p271 et Couvreur, Pharmaceutical Research, 1994, p 1270.

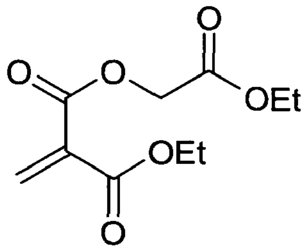

- les dérivés itaconate comme :

l'itaconate de diméthyle décrit dans la référence suivante par Bachrach, European Polymer Journal, 1976, p563

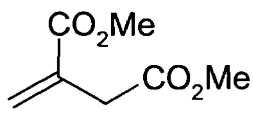

- les dérivés α-(methylsulfonyl)acrylates de méthyle (K), α-(methylsulfonyl)acrylates de d'éthyle (L), α-(tert-butylsulfonyl)acrylates de méthyle (M), α-(methylsulfonyl)acrylates de tert-butyle (N), α-( tert-butylsulfonyl)acrylates de tert-butyle (O) par Gipstein, J.Org.Chem, 1980, p1486 et
- les dérivés 1,1-bis-(methylsulfonyl)ethylene (P), 1-acetyl-1-methyl sulfonyl ethylene (Q), α-(methylsulfonyl) vinyl sulfonate de methyle (R), α-methylsulfonylacrylonitrile (S) par Shearer, US patent US2748050 :

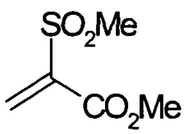
(K)

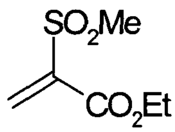
(L)

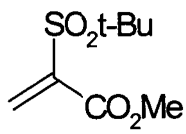
(M)

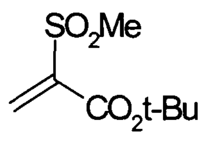
(N)

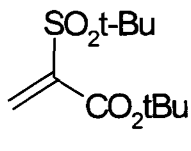
(O)

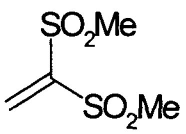
(P)

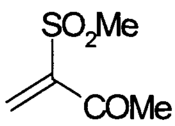
(Q)

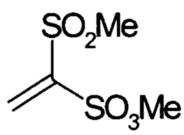
(R)

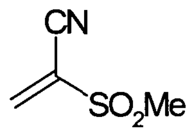
(S)

Dans un autre mode de réalisation particulier, le (ou les) monomère(s) électrophile(s) présent(s) dans la composition de l'invention peu(ven)t être choisi(s) parmi :

- La méthyl vinyl sulfone et la phényl vinyl sulfone notamment décrits par Boor , J.Polymer Science, 1971, p249

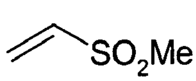

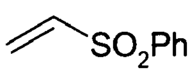

- le phényl vinyl sulfoxide notamment décrit par Kanga, Polymer preprints (ACS, Divison of Polymer Chemistry), 1987, p322

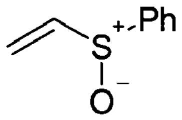

- le 3-methyl-N-(phenylsulfonyl)-1-aza-1,3-butadiene notamment décrit par Bonner, Polymer Bulletin, 1992, p517

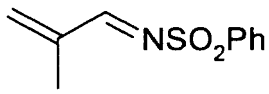

- les monomères acrylamides tels que le :

  n-propyl-n-(3-trüsopropoxysilylpropyl)acrylamide et le n-propyl-n-(3-triethoxysilylpropyl)acrylamide notamment décrits par Kobayashi, Journal of Polymer Science, Part A: Polymer Chemistry, 2005, p2754.

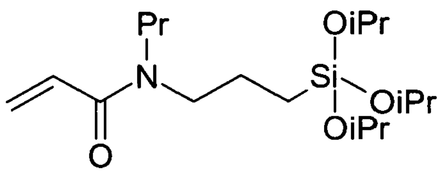 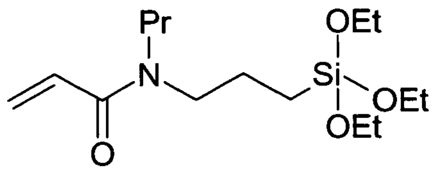

- les monomères acrylates tels que :

  l'acrylate de 2-hydroxyethyl et le méthacrylate de 2-hydroxyethyl
  l'acrylate de n-butyl
  l'acrylate de tertio-butyle

- les monomères itaconimide choisis parmi :

  le n-butyl itaconimide (F) , n-(4-tolyl) itaconimide (G) , n-(2-ethylphenyl) itaconimide (H) , N-(2,6-diethylphenyl) itaconimide (I) notamment décrits dans la référence suivante par Wanatabe, J.Polymer Science : Part A :Polymer chemistry, 1994, p2073

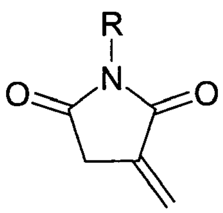

R= Bu (F), 4-ethyphenyl (H), 2,6-diethylkphenyl (I)

Selon un mode préféré de réalisation, le monomère électrophile est un monomère de la famille des cyanoacrylates et leurs dérivés de formule (B) :

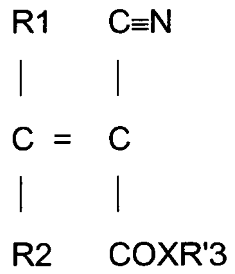

(B)

X désignant NH, S ou O,
R1 et R2 ayant les mêmes significations que précédemment, de préférence R1 et R2 représentant un atome d'hydrogène,
R'3 représentant un atome d'hydrogène ou R tel que défini à la formule (A).
De préférence, X désigne O.

A titre de composés de formule (B), on peut citer les monomères :

a) appartenant à la famille des 2-cyanoacrylates de polyfluoroalkyle en C1-C20 tels que:

l'ester 2,2,3,3-tétrafluoropropylique de l'acide 2-cyano-2-propénoïque de formule :

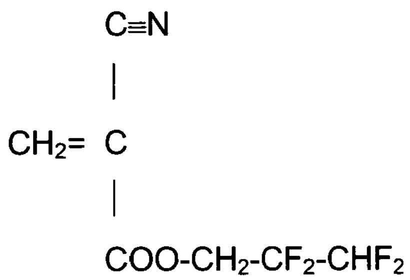

ou encore l'ester 2,2,2-trifluoroéthylique de l'acide 2-cyano-2-propénoïque de formule :

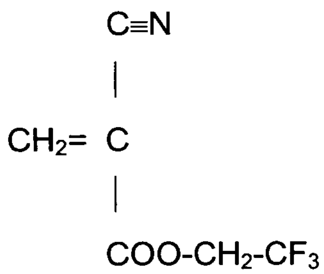

b) les cyanoacrylates d'alkyle en C1-C10 ou d'(alcoxy en C1-C4) alkyle en C1-C10.
On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle,
c) les dérivés benzylidène malononitrile de formule

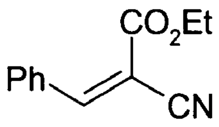

le 2-(4-chloro-benzylidène)-malononitrile de formule

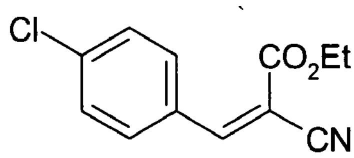

et leurs mélanges.

Dans le cadre de l'invention, on préfère utiliser les monomères b).

Selon un mode de réalisation préféré, le ou les monomères cyanoacrylates sont choisis parmi les cyanoacrylates d'alkyle en C6-C10.

Les monomères particulièrement préférés sont les cyanoacrylates d'octyle de formule F et leurs mélanges :

$$C\equiv N$$
$$|$$
$$CH2= C \qquad\qquad (F)$$
$$|$$
$$COO\text{-}R'_3$$

dans laquelle : $R'_3$ est choisi parmi $=\text{-}(CH_2)_7\text{-}CH_3$,
$\text{-}CH(CH_3)\text{-}(CH_2)_5\text{-}CH_3$,
$\text{-}CH_2\text{-}CH(C_2H_5)\text{-}(CH_2)_3\text{-}CH_3$,
$\text{-}(CH_2)_5\text{-}CH(CH_3)\text{-}CH_3$,
$\text{-}(CH_2)_4\text{-}CH(C_2H_5)\text{-}CH_3$;

Le monomère cyanoacrylate particulièrement préféré selon l'invention est l'octyl 2-cyanoacrylate, linéaire ou ramifié, vendu sous la référence commerciale RITELOK CDN 1064 par la société Chemence.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Les monomères cyanoacrylates de formule (B) selon la présente invention peuvent être synthétisés selon les méthodes connues décrites dans la technique. En particulier, les monomères cyanoacrylates peuvent être synthétisés selon l'enseignement des brevets US 3 527 224, US 3 591 767, US 3 667 472, US 3 995 641, US 4 035 334 et US 4 650 826.

Le monomère électrophile, et en particulier le monomère cyanoacrylate de formule (B), notamment l'octyl 2-cyanoacrylate peut être présent en une teneur allant de 0,1 et 99 % en poids, par rapport au poids total de la composition le comprenant, de préférence allant de 1 et 90 % en poids, de préférence allant de 1 à 70% en poids, de préférence allant de 1 à 60% en poids, de préférence allant de 1 à 50% en poids, de préférence allant de 1 à 40% en poids, et plus préférentiellement allant de 1 à 30% en poids.

Le polymère en dispersion utilisé dans la présente invention peut être de toute nature. On peut ainsi employer un polymère radicalaire, un polycondensat, voire un polymère d'origine naturelle et leurs mélanges. Ce polymère peut être choisi par l'homme du métier en fonction de ses propriétés et selon l'application ultérieure souhaitée pour la composition. De préférence le polymère utilisé est filmifiable. Il est toutefois possible d'utiliser un polymère non filmifiable.

Par polymère non filmifiable, on entend un polymère non capable de former, seul, un film isolable. Ce polymère permet, en association avec un composé non volatil du type huile, de former un dépôt continue et homogène sur la

peau et les muqueuses comme les lèvres.

Un avantage de l'utilisation d'une dispersion de particules dans une composition de l'invention est que les particules restent à l'état de particules élémentaires, sans former d'agglomérats, dans la phase grasse, ce qui ne serait pas le cas avec des particules minérales de taille nanométrique. Un autre avantage de la dispersion de polymère est la possibilité d'obtenir des compositions très fluides (de l'ordre de 130 centipoises), même en présence d'un taux élevé de polymère.

Encore un autre avantage d'une telle dispersion est qu'il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur les matières kératiniques. Ceci ne serait pas possible avec des pigments sous forme particulaire, leur constitution ne permettant pas de moduler la taille moyenne des particules.

La composition selon l'invention comprend donc avantageusement une ou plusieurs dispersions stables de particules généralement sphériques d'un ou plusieurs polymères, dans une phase grasse liquide physiologiquement acceptable. Ces dispersions peuvent notamment se présenter sous forme de nanoparticules de polymères en dispersion stable dans ladite phase grasse. Les nanoparticules sont de préférence d'une taille comprise entre 5 et 600 nm, étant donné qu'au-delà d'environ 600 nm, les dispersions de particules deviennent beaucoup moins stables.

Encore un avantage de la dispersion de polymère de la composition de l'invention est la possibilité de faire varier la température de transition vitreuse (Tg) du polymère ou du système polymérique (polymère plus additif du type plastifiant), et de passer ainsi d'un polymère dur à un polymère plus ou moins mou, permettant de régler les propriétés mécaniques de la composition en fonction de l'application envisagée.

Il est possible d'utiliser des polymères filmifiables, de préférence ayant une Tg basse, inférieure ou égale à la température de la peau. On obtient ainsi une dispersion qui peut filmifier lorsqu'elle est appliquée sur un support, ce qui n'est pas le cas lorsqu'on utilise des dispersions de pigments minéraux selon l'art antérieur.

Les polymères en dispersion utilisables dans la composition de l'invention ont de préférence un poids moléculaire de l'ordre de 2000 à 10000000, de préférence entre 3000 et 800000 et encore plus préférentiellement entre 4000 et 500000, et une Tg de -150°C à 300°C. De préférence, la température de transition vitreuse est comprise entre -150°C et 0°C, et encore plus préférentiellement entre -100°C et -20°C.

Lorsque le polymère présente une température de transition vitreuse trop élevée pour l'application souhaitée, on peut lui associer un plastifiant de manière à abaisser cette température du mélange utilisé. Le plastifiant peut être choisi parmi les plastifiants usuellement utilisés dans le domaine d'application et notamment parmi les composés susceptibles d'être des solvants du polymère.

Parmi les polymères filmifiables, on peut citer des homopolymères ou des copolymères radicalaires, acryliques ou vinyliques, de préférence ayant une Tg inférieure ou égale à 40°C et notamment les acrylates de méthyle éventuellement copolymérisés avec l'acide acrylique.

Parmi les polymères non filmifiables, on peut citer des homopolymères ou copolymères radicalaires, vinyliques ou acryliques, éventuellement réticulés, ayant de préférence une Tg supérieure ou égale à 40°C, tels que le polyméthacrylate de méthyle, le polystyrène ou le polyacrylate de tertiobutyle.

De façon non limitative, les polymères en dispersion de l'invention peuvent être choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides, polyesters à chaîne grasse, alkydes ; polymères ou copolymères acryliques et/ou vinyliques ; copolymères acryliques-silicone ; polyacrylamides ; polymères siliconés comme les polyuréthanes ou acryliques siliconés, polymères fluorés et leurs mélanges.

Le polymère utilisé selon l'invention peut être un homopolymère ou un copolymère.

Les polymères dont la température de transition vitreuse est inférieure ou égale à 0°C peuvent être un homopolymère ou un copolymère qui de préférence est issue en totalité ou en partie d'un ou de plusieurs monomères :

- qui sont tel(s) que les homopolymères ou copolymères sont préparés à partir de monomères dont les températures de transition vitreuse sont inférieures ou égales à -20°C, ou
- qui sont tels que les copolymères sont préparés à partir de monomères dont l'un d'entre eux au moins est un monomère dont l'homopolymère possède une Tg inférieure à -20°C et au moins un monomère additionnel dont la Tg de l'homopolymère correspondant est supérieure à -20°C en quantité telle que la Tg globale du système est inférieure à -20°C.

De façon non exhaustive, on peut citer comme monomères exploitables pour la synthèse de la dispersion de polymère de l'invention :

- les acrylates de formule CH2=CHCOOR1, R1 représentant un groupe alkyle ayant de 1 à 28 atomes de carbone, linéaire ou ramifié, dans lequel se trouve(nt) éventuellement intercalé(s) un ou plusieurs hétéroatomes choisis parmi

O, N, S, ledit groupe alkyle pouvant en outre être éventuellement substitué par un ou plusieurs substituants choisis parmi les groupes hydroxyle et les atomes d'halogène (Cl, Br, I et F) et interrompus par un ou plusieurs hétératomes choisis parmi O, N, S et P ; des exemples de groupes R1 sont les groupes, éthyle, propyle, butyle, isobutyle, hexyle, éthylhexyle, octyle, lauryle, isooctyle, isodécyle, hydroxyéthyle, hydroxypropyle. R1 peut aussi désigner un groupement polyoxyéthylène comprenant de 5 à 30 motifs d'oxyde d'éthylène ; un autre exemple de R1 pour les acrylates sont les groupes alkyle en C1-C12 - POE (polyoxyéthylène), avec répétition du motif oxyéthylène de 5 à 30 fois, par exemple méthoxy-POE, soit les groupes R1 = R-(OC2H4)n-, avec R = alkyle en C1-C12, et n = 5 à 30 ; des exemples de tels esters sont l'acrylate de méthyle, l'acrylate d'éthyle, l'acrylate de n butyle, l'acrylate de 2-éthylhexyle ;

- les méthacrylates de formule : CH2=CR2COOR1, R1 représentant un groupe alkyle en C5-C28, linéaire ou ramifié, dans lequel se trouve(nt) éventuellement intercalé(s) un ou plusieurs hétéroatomes choisis parmi 0, N et S, ledit groupe alkyle pouvant en outre être éventuellement substitué par un ou plusieurs substituants choisis parmi les groupes hydroxyle et les atomes d'halogènes (Cl, Br, I, F) ; des exemples de groupes R2 sont les groupes octyle, lauryle, isooctyle, isodécyle, dodécyle, POE (polyoxyéthylène avec répétition du motif oxyéthylène de 5 à 30 fois) et alkyl (C1-C30) - POE (avec répétition du motif oxyéthylène de 5 à 30 fois) ; des exemples de tels esters sont la méthacrylate de 2-éthylhexyle ;
- les esters de vinyle de formule : R3 CH = CH2 où R3 représente un groupe acyloxy en C2-C 12, linéaire ou ramifié ; des exemples de tels esters de vinyle sont le butyrate de vinyle, l'éthylhexanoate de vinyle, le néononanoate de vinyle, le néododécanoate de vinyle, et l'acétate de vinyle ;
- les éthers de vinyle CH2=CH-OR4 avec R4 désignant un groupe alkyle en C1-C12, tels que l'éther de vinyle, le méthyl éther vinylique, l'éthyl vinyl éther ;
- les composés vinyliques de formule :CH2 = CH R4, où R4 est un groupe hydroxyle; un groupe cycloalkyle en C3-C8 ; un groupe aryle en C6-C20 ; un groupe aralkyle en C7-C30 (groupe alkyle en C1 à C4) ; un groupe hétérocyclique de 4 à 12 chaînons contenant un ou plusieurs héréatomes choisis parmi O, N, et S ; un groupe hétérocyclylalkyle (alkyle en C1-C4) tel qu'un groupe furfuryle ; lesdits groupes cycloalkyle, aryle, aralkyle, hétérocyclique, ou hétérocyclylalkyle pouvant être éventuellement substitués par un ou plusieurs substituants choisis parmi les groupes hydroxyles, les atomes d'halogène, et les groupes alkyles en C1-C4, linéaires ou ramifiés, pouvant être éventuellement interrompus par un ou plusieurs hétéroatomes choisis parmi 0, N, S et P ; des exemples de monomères vinyliques sont le vinylcyclohexane, et le styrène ;
- les acrylates de formule : CH2 = CHCOOR5, où R5 est un groupe cycloalkyle en C3-C8 ; un groupe aryle en C6-C20 ; un groupe aralkyle en C7-C30 (groupe alkyle en C1-C4) ; un groupe hétérocyclique de 4 à 12 chaînons contenant un ou plusieurs hétéroatomes choisis parmi O, N, et S ; un groupe hétérocyclylalkyle (alkyl de C1 à C4), tel qu'un groupe furfuryle ; lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés pouvant être éventuellement interrompus par un ou plusieurs hétéroatomes choisis parmi 0, N, S et P ; des exemples de monomères acrylate de ce type sont les acrylates de butylcyclohexyle, de butylbenzyle, de furfuryle et d'isobornyle ;
- les méthacrylates de formule :CH2 = C(CH3)COOR6, où R6 est un groupe alkyle en C1-C4, linéaire ou ramifié, tel qu'un groupe méthyle, éthyle, propyle ou isobutyle, ledit groupe alkyle pouvant en outre être éventuellement substitué par un ou plusieurs substituants choisis parmi les groupes hydroxyle et les atomes d'halogène (C1, Br, I et F) ; un groupe cycloalkyle en C3-C8 ; un groupe aryle en C6-C20 ; un groupe aralkyle en C7-C30 (groupe alkyle en C1-C4) ; un groupe hétérocyclique de 4 à 12 chaînons contenant un ou plusieurs hétéroatomes choisis parmi O, N, et S ; un groupe hétérocyclylalkyle (alkyle de 1 à 4 C), tel qu'un groupe furfuryle ; lesdits groupes alkyle, cycloalkyle, aryle, aralkyle, ou hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifié pouvant être éventuellement interrompus par un ou plusieurs hétéroatomes choisis parmi O, N, S et P ; des exemples de monomères méthacrylate sont les méthacrylates de méthyle, d'éthyle, de n-butyle, d'isobutyle, de tertiobutyle, butylcyclohexyle, de butylbenzyle, de méthoxyéthyle de méthoxypropyle et d'isobornyle ;
- les (méth)acrylamides de formule : CH2=CR8NHCOR7, où R7 représente un atome d'hydrogène ou un groupe alkyle en C1-C18, linéaire ou ramifié, tel qu'un groupe n-butyle, t-butyle, isopropyle, isohexyle, isooctyle, ou isononyle, et où R8 désigne un atome d'hydrogène ou un radical méthle. Des exemples de monomères (méth)acrylamide sont le N-butylacrylamide, le Nbutylacrylamide, le Nisopropylacrylamide, le N,Ndiméthylacrylamide, le N,N dibutylacrylamide et la Noctylacrylamide, et la Noctadécylacrylamide ;
- les monomère hydrophiles anioniques tels que l'acide acrylique, l'acide méthacrylique. Le polymère peut être utilisé neutralisé par un neutralisant tel que : une base minérale, (LiOH, NaOH, KOH, Ca(OH)2, NH4OH), une base organique, par exemple une amine primaire, secondaire ou tertiaire, l'amine pouvant comporter ou non des substituants comme l'amino-2-méthyl-2-propanol, et les formes salifiées ou quaternisées de ceux-ci ;
- les monomère hydrophiles cationiques tels que les monomères comportant des fonctions amines tertiaires, comme

par exemple : dimethylaminoethylmethacrylate MADAME (0.97), DMAPMA (N,N-diméthylaminopropylméthacrylamide, vinylpyridine(1.20), vinylimidazole (0.96) et neutralisés ou non par des acides pouvant être des sels d'acides minéraux, tels que l'acide sulfurique ou l'acide chlorhydrique, ou des sels d'acides organiques. Ces acides organiques peuvent comporter un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. Un exemple d'acide à groupe alkyle est l'acide acétique CH3COOH. Un exemple de polyacide est l'acide téréphtalique. Des exemples d'hydroxyacides sont l'acide citrique et l'acide tartrique ;

- les monomère hydrophiles cationiques tels que les monomères comportant des fonctions amines quaternaires tels que par exemple le le chlorure de méthacryloyloxyéthyltriméthylammonium (MADQUAT), le chlorure de méthacrylamidopropyltriméthylammonium (MAPTAC) ;
- la vinylpyrrolidone.

Les particules de polymère stabilisées peuvent être présentes dans la composition à des teneurs comprises entre 0,0001 et 30% en poids, de préférence entre 0,001 et 20%, et encore de préférence entre 0,01 et 10% en poids du poids total de la composition.

La phase grasse liquide de la composition peut être constituée de toute huile cosmétiquement ou dermatologiquement acceptable, et de façon générale physiologiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

Le milieu de dispersion des polymères de l'invention constitue tout ou partie de cette phase grasse liquide.

Par "phase grasse liquide", on entend tout milieu non aqueux liquide à température ambiante et pression atmosphérique. Cette phase grasse peut contenir une phase grasse liquide volatile et/ou une phase grasse non volatile.

Par "phase grasse volatile", on entend tout milieu non aqueux susceptible de s'évaporer de la peau ou des lèvres, en moins d'une heure. Cette phase volatile comporte notamment des huiles ayant une pression de vapeur, à température ambiante et pression atmosphérique allant de 10-3 à 300mm de Hg.

La phase grasse liquide totale de la composition peut représenter de 5 % à 97,90 % du poids total de la composition et de préférence de 20 à 85 %. La partie non volatile peut représenter de 0 à 80 % du poids total de la composition et mieux de 1 à 50% .

Comme phase grasse liquide utilisable dans l'invention, on peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les polydiméthylsiloxanes (PDMS), éventuellement phénylées telles que les phényltriméthicones ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

Avantageusement, on peut utiliser une ou plusieurs huiles volatiles à température ambiante (20°C). Ces huiles volatiles sont favorables à l'obtention d'un film à propriétés "sans transfert" total. Après évaporation de ces huiles, on obtient un dépôt filmogène souple, non collant sur la peau ou les muqueuses, suivant respectivement le mouvements de la peau ou des lèvres, sur lesquelles la composition est appliquée. Ces huiles volatiles facilitent, en outre, l'application de la composition sur la peau, les muqueuses et les phanères. Elles peuvent être hydrocarbonées ou siliconées comportant éventuellement des groupements alkyle ou alkoxy, pendants ou en bout de chaîne siliconée.

Comme huile volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone ainsi que les isoparaffines en C8-C16. Ces huiles volatiles représentent notamment de 20 à 97,90 % du poids total de la composition, et mieux de 30 à 75 %.

Comme huile volatile utilisable dans l'invention, on peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane ou les isoparaffines en $C_8$-$C_{16}$ telles que les 'ISOPARs', les PERMETYLs et notamment l'isododécane.

Dans un mode particulier de réalisation de l'invention, on choisit la phase grasse liquide dans le groupe

comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

Le paramètre de solubilité global δ global selon l'espace de solubilité de HANSEN est défini dans l'article "Solubility parameter values" de Eric A. Grulke de l'ouvrage "Polymer Handbook" 3ème édition, Chapitre VII, pages 519-559 par la relation :

$$\delta = ( d_D^2 + d_P^2 + d_H^2)^{1/2} ,$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.). La définition des solvants dans l'espace de solubilité tridimensionnel selon HANSEN est donnée dans l'article de C. M. HANSEN : "The three dimensional solubility parameters" J. Paint Technol. 39, 105 (1967).

Parmi les phases grasses liquides ayant un paramètre de solubilité global selon l'espace de solubilité de HANSEN, inférieur ou égal à 17 $(MPa)^{1/2}$, on peut citer des huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle. On peut également citer les hydrocarbures et notamment des huiles de paraffine, de vaseline, ou le polyisobutylène hydrogéné, l'isododécane, ou encore les 'ISOPARs', isoparaffines volatiles. On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyle, thiol et/ou amine, et les huiles siliconées volatiles, notamment cycliques. On peut également citer les solvants, seuls ou en mélange, choisis parmi (i) les esters linéaires, ramifiés ou cycliques, ayant plus de 6 atomes de carbone, (ii) les éthers ayant plus de 6 atomes de carbone, (iii) les cétones ayant plus de 6 atomes de carbone. Par monoalcools ayant un paramètre de solubilité global selon l'espace de solubilité de HANSEN inférieur ou égal à 20 $(MPa)^{1/2}$, on entend les alcools gras aliphatiques ayant au moins 6 atomes de carbone, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Comme milieu non aqueux, on peut aussi utiliser ceux décrits dans le document FR-A-2 710 646 de L.V.M.H.

Le choix du milieu non aqueux est effectué par l'homme du métier en fonction de la nature des monomères constituant le polymère en dispersion et/ou de la nature du stabilisant, comme indiqué ci-après.

La dispersion de polymère peut être fabriquée comme décrit dans le document EP-A-749747. La polymérisation peut être effectuée en dispersion, c'est-à-dire par précipitation du polymère en cours de formation, avec protection des particules formées avec un stabilisant.

On prépare donc un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie est une huile volatile, on peut directement effectuer la polymérisation dans

ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

Les particules de polymère sont stabilisées en surface, au fur et à mesure de la polymérisation, grâce à un stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide polyhydroxystéarique : copolymères à base d'acrylates ou de méthacrylates d'alcools en $C_1$-$C_4$, et d'acrylates ou de méthacrylates d'alcools en $C_8$-$C_{30}$.

Comme copolymères blocs greffés ou séquencés comprenant au moins un bloc de type polyorganosiloxane et au moins un bloc d'un polymère radicalaire, on peut citer les copolymères greffés de type acrylique/silicone qui peuvent être employés notamment lorsque le milieu non aqueux est siliconé.

Comme copolymères blocs greffés ou séquencés comprenant au moins un bloc de type polyorganosiloxane et au moins d'un polyéther, on peut utiliser les copolyol diméthicones tels que ceux vendu sous la dénomination "DOW CORNING 3225C" par la société DOW CORNING, les lauryl méthicones tels que ceux vendu sous la dénomination "DOW CORNING Q2-5200 par la société "DOW CORNING".

Comme copolymères blocs greffés ou séquencés comprenant au moins un bloc résultant de la polymérisation d'au moins un monomère éthylénique, à une ou plusieurs liaisons éthyléniques éventuellement conjuguées, et au moins un bloc d'un polymère vinylique, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'KRATON' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS).

Comme copolymères blocs greffés ou séquencés comprenant au moins un bloc résultant de la polymérisation d'au moins un monomère éthylénique et au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique et au moins un bloc d'un polyéther, on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères d'acrylates ou de méthacrylates d'alcools en C1-C4, et d'acrylates ou de méthacrylates d'alcools en C8-C30. On peut en particulier citer le copolymère méthacrylate de stéaryle/méthacrylate de méthyle.

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé,

de manière à avoir une meilleure activité interfaciale. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque la phase grasse liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alcools en C1-C4, et d'acrylates ou de méthacrylates d'alcools en C8-C30,
- (c) les copolymères blocs greffés ou séquencés comprenant au moins un bloc résultant de la polymérisation d'au moins un monomère éthylénique, à liaisons éthyléniques conjuguées,
  et au moins un bloc d'un polymère vinylique ou acrylique ou d'un polyéther ou d'un polyester, ou leurs mélanges.

De préférence, on utilise des polymères dibloc comme agent stabilisant.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges. Les groupements alkyle désignent de préférence des groupements ayant 1 à 6 atomes de carbone.

On peut aussi utiliser des acides minéraux ou organiques, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Les compositions conformes à l'invention peuvent également contenir au moins un agent utilisé habituellement en cosmétique, tel que, par exemple, des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, des gaz propulseurs, des épaississants minéraux ou organiques tels que le benzylidène sorbitol, les N acylaminoacides. Ces agents peuvent être éventuellement encapsulés. La capsule peut être de type polycyanoacrylate.

Le procédé de traitement des cheveux conforme à l'invention consiste à appliquer la composition décrite ci-dessus sur les matières kératiniques, et en particulier en présence d'un agent nucléophile avec ou sans chauffage.

De préférence, cet agent nucléophile est l'eau. Cette eau peut être apportée par une humidification préalable.

Il est également possible, afin de moduler la cinétique de réaction, de préalablement humidifier les matières kératiniques à l'aide d'une solution aqueuse dont le pH a été ajusté à l'aide d'une base, d'un acide ou d'un mélange acide/base. L'acide et/ou la base peuvent être inorganique ou organique.

Ces deux opérations peuvent aussi être effectuées après application de la composition.

Il est également possible de moduler la cinétique de polymérisation par voie anionique en pré-imprégnant les matières kératiniques à l'aide d'un agent nucléophile. L'agent nucléophile peut être utilisé pur, en solution, sous forme d'une émulsion, ou être encapsulé.

Les agents nucléophiles susceptibles d'initier la polymérisation anionique sont des systèmes connus en eux-mêmes, capables de générer un carbanion au contact d'un agent nucléophile. Les agents nucléophiles peuvent notamment être constitutés par les ions hydroxyles contenus dans l'eau.

L'agent nucléophile peut être un composé moléculaire, un oligomère, un dendrimère ou un polymère possédant des fonctions nucléophiles. De façon non limitative, on peut citer comme fonctions nucléophiles les fonctions : $R_2N^-$,

$NH_2^-$, $Ph_3C^-$, $R_3C^-$, $PhNH^-$, pyridine, $ArS^-$, R-C≡$C^-$, $RS^-$, SH, $RO^-$, $R_2NH$, $ArO^-$, $N_3^-$, $OH^-$, $ArNH_2$, $NH_3$, $I^-$, $Br^-$, $Cl^-$, $RCOO^-$, $SCN^-$, ROH, RSH, $NCO^-$, $CN^-$, $NO_3^-$, $ClO_4^-$ et $H_2O$, Ph représentant le groupe phényle ; Ar représentant un groupe aryle et R représentant un groupe alkyle en $C_1$-$C_{10}$.

Les agents nucléophiles particulièrement préférés sont les ions hydroxyles, notamment ceux présents dans l'eau.

L'eau contenant les ions hydroxyles nucléophiles peut être en particulier l'eau apportée par une source extérieure, par exemple un brumisateur, ou bien encore de l'eau apportée par une seconde composition.

Selon un mode préféré de réalisation, la composition comprenant le monomère électrophile est exempte d'agent nucléophile.

Selon un autre mode préféré de réalisation, l'agent nucléophile est apporté par une seconde composition, appliquée sur ou sous le dépôt formé par l'application de la composition comprenant le monomère électrophile.

Il est également possible, afin de moduler la cinétique de réaction, de préalablement humidifier la fibre à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant la fibre à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie de la fibre par transformation chimique de la matière kératinique.

A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:

- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylates tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions peut être suivie ou non d'un rinçage. Ces compositions peuvent se présenter sous des formes diverses, telles que de lotion, de spray, de mousse et être appliquées sous forme de shampooing ou d'après-shampooing.

La composition selon l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Le procédé peut comprendre une étape d'application sur les matières kératiniques de particules de polymère

dispersées dans une phase grasse liquide et stabilisées en surface par un agent stabilisant, et une étape d'application sur les matières kératiniques d'au moins un monomère électrophile, l'ordre des étapes étant indifférent et un éventuel rinçage séparant ces deux étapes.

Dans un mode de réalisation particulier, l'application des particules de polymère est réalisée avant l'application du ou des monomères électrophiles.

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence de 20 à 80°C, ce qui n'empêche pas de terminer l'application par un séchage au casque, un brushing ou passage au fer plat ou à friser.

Les exemples qui suivent sont destinés à illustrer l'invention.

**Exemples 1 à 7**

Les pourcentages sont exprimés en pourcentage en poids de matière active.

| | | | | | | | |
|---|---|---|---|---|---|---|---|
| Dispersion non aqueuse de polymère [1] | 5% | - | - | - | - | - | - |
| Dispersion non aqueuse de polymère [2] | - | 5% | - | - | - | - | - |
| Dispersion non aqueuse de polymère [3] | - | | 5% | - | - | - | - |
| Dispersion non aqueuse de polymère [4] | - | - | - | 5% | - | - | - |
| Dispersion non aqueuse de polymère [5] | - | - | - | | 5% | | |
| Dispersion non aqueuse de polymère [6] | - | - | - | - | - | 5% | |
| Dispersion non aqueuse de polymère [7] | - | - | - | - | - | - | 5% |
| 2-cyanoacrylate de 2-octyle [8] | 10% | 10% | 10% | 10% | 10% | 10% | 10% |
| poly diméthylsiloxane alpha-omega dihydroxyle/ cyclopenta diméthylsiloxane (14.7/85.3) [9] | 40% | 40% | 40% | 40% | 40% | 40% | 40% |
| Cyclopentadiméthylsiloxane [10] | qsp 100% | qsp 100% | qsp 100% | qsp 100% | qsp 100% | qsp 100% | qsp 100% |

[1] Dispersion de polyacrylate de 2 ethylhexyle à 30% dans de la cyclopentadimethylsiloxane. La dispersion est stabilisée par 2% de polydimethylsiloxane à greffons perfluorononyle (PECOSIL FSH 150 commercialisé par la société SEPPIC).

[2] Dispersion de polyacrylate de nonyle à 30% dans de la cyclopentadimethylsiloxane. La dispersion est stabilisée par 5% de polydimethylsiloxane oxyéthylènée (KF-6017 commercialisé par la société SHIN ETSU).

[3] Dispersion de copolymère d'acrylate de 2 ethylhexyle et de méthacrylate de dodecafluoroheptyl (97/3) à 30 % dans de la cyclopentadimethylsiloxane. La dispersion est stabilisée par 5% de polydimethylsiloxane oxyéthylènée (KF-6017 commercialisé par la société SHIN ETSU).

[4] Dispersion de copolymère acrylate de méthyle et acide acrylique (90/10) à 30 % dans de la cyclopentadimethylsiloxane. La dispersion est stabilisée par 2% de polydimethylsiloxane à greffons perfluorononyle (PECOSIL FSH 150 commercialisé par la société SEPPIC).

[5] Dispersion de copolymère d'acrylate de 2 ethylhexyle et de dimethyaminoethylmethacrlate neutralisé par de l'acide 2ethyl caproïque (85/7.5/7.5) à 30 % dans de la cyclopentadimethylsiloxane. La dispersion est stabilisée par 2% de polydimethylsiloxane à greffons perfluorononyle (PECOSIL FSH 150 commercialisé par la société SEPPIC).

[6] Dispersion de copolymère acrylate de méthyle et acide acrylique (90/10) à 30 % dans de la cyclopentadimethylsiloxane. Le polymère est plastifié par 2.5% de propylène glycol introduit avant amorçage de la polymérisation. La dispersion est stabilisée par 2% de polydimethylsiloxane à greffons perfluorononyle (PECOSIL FSH 150 commercialisé par la société SEPPIC).
[7] Dispersion de copolymère acrylate de méthyle (90% molaire) et acide acrylique (10% molaire) à 30 % dans de la cyclopentadimethylsiloxane. Le polymère est plastifié par 2.5% du monomethylether de tripropylène glycol introduit avant amorçage de la polymérisation. La dispersion est stabilisée par 2% de polydimethylsiloxane à greffons perfluorononyle (PECOSIL FSH 150 commercialisé par la société SEPPIC)
[8] RITE LOK CON895, commercialisé par la Société CHEMENCE.
[9] commercialisé par Dow Corning sous le nom DC 1501 Fluid
[10] commercialisé par Dow Corning sous le nom DC 245 Fluid

**Exemples 8 et 9**

Dispersion A : on prépare une dispersion de copolymère non réticulé d'acrylate de méthyle et d'acide acrylique dans un rapport 95/5, dans l'isododécane, selon la méthode de l'exemple 1 du document EP-A-749 746, en remplaçant l'heptane par l'isododécane. on obtient ainsi une dispersion de particules de poly(acrylate de méthyle/acide acrylique) stabilisées en surface dans l'isododécane par un copolymère dibloc séquencé polystyrène/coply(éthylène-propylène) vendu sous le nom de Kraton G1701, ayant un taux de matière sèche de 25% en poids.
Formule B (pour 100g de formule)
20 g de dispersion A
10 g de 2-octylcyanoacrylate
35g de Dow Corning 245 Fluid
35g de Dow Corning 1501 Fluid
Formule C
90 g de dispersion A
10g de 2-octylcyanoacrylate
Les cheveux ont été lavés au shampooing et essorés. Les formule B (exemple 8) ou C (exemple 9) ont été appliquées sur une mèche à raison de 0,16g de formule pour 1 g de cheveux. Les mèches ont été malaxées avec les doigts pendant 1 à 2 minutes, puis peignées. Elles ont été séchées au casque pendant 30 minutes, à une température de l'ordre de 40°C.
Dans les deux cas, les cheveux sont ensuite enrobés et cet enrobage est rémanent à au moins 10 shampooings. Les formules confèrent du volume à la chevelure et cet effet est rémanent.

## Revendications

1. Composition cosmétique, notamment capillaire, **caractérisée en ce qu'**elle comprend au moins un monomère électrophile, une phase grasse liquide, et des particules de polymère dispersées dans la phase grasse liquide et stabilisées en surface par un agent stabilisant.

2. Composition selon la revendication 1, **caractérisée en ce que** le ou les monomères électrophiles répondent à la formule :

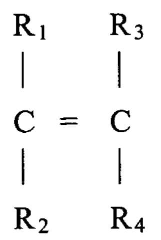

(A)

dans laquelle :

$R_1$ et $R_2$ désignent chacun, indépendamment l'un de l'autre, un groupe peu ou non électro-attracteur choisi

parmi :

- un atome d'hydrogène,
- un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
- un résidu polyorganosiloxane modifié ou non,
- un groupement polyoxyalkylène,

$R_3$ et $R_4$ désignent chacun, indépendamment l'un de l'autre, un groupe électro-attracteur choisi parmi les groupements $-N(R)_3^+$, $-S(R)_2^+$, $-SH_2^+$, $-NH_3^+$, $-NO_2$, $-SO_2R$, -C≡N, -COOH, -COOR, -COSR, $-CONH_2$, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en $C_1$- $C_4$, les groupements aryle et aryloxy, R désigne un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un radical alkyle en $C_1$-$C_{10}$.

3. Composition selon la revendication 2, **caractérisée en ce que** le ou les monomères électrophiles sont choisis parmi les composés de formule :

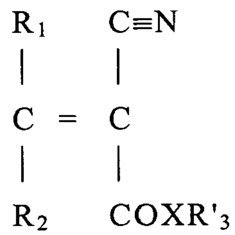

(B)

dans laquelle
X désigne NH, S, O,
$R_1$ et $R_2$ sont tels que définis dans la revendication 3,
$R'_3$ désigne un atome d'hydrogène ou un radical R tel que défini dans la revendication 6.

4. Composition selon la revendication 3, **caractérisée en ce que** le
ou les monomères sont choisis parmi les 2-cyanoacrylates de polyfluoroalkyle en $C_1$-$C_{20}$, les cyanoacrylates d'alkyle en ($C_1$-$C_{10}$) ou d'(alcoxy en $C_1$-$C_4$)(alkyle en $C_1$-$C_{10}$).

5. Composition selon la revendication 4, **caractérisée en ce que** le
ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

6. Composition selon la revendication 3, **caractérisée en ce que** le
ou les monomères électrophiles répondent à la formule (IIc) :

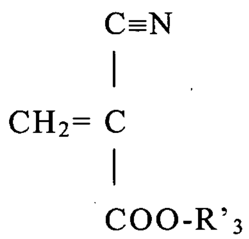 (F)

dans laquelle : $R'_3$=$-(CH_2)_7-CH_3$,
$-CH(CH_3)-(CH_2)_5-CH_3$,
$-CH_2-CH(C_2H_5)-(CH_2)_3-CH_3$,
$-(CH_2)_5-CH(CH_3)-CH_3$,
$-(CH_2)_4-CH(C_2H_5)-CH_3$,

7. Composition selon l'une quelconque des revendications précédentes, **caractérisée en ce que** le monomère est présent dans la composition à des teneurs comprises entre 0,1 et 99% en poids, de préférence entre 1 et 90%, et encore de préférence entre 1 et 30% en poids du poids total de la composition.

8. Composition selon l'une quelconque des revendications précédentes, **caractérisée en ce que** la composition comprend des inhibiteurs de polymérisation.

9. Composition selon la revendication 8, **caractérisée en ce que** les inhibiteurs sont des inhibiteurs de polymérisation anioniques et/ou radicalaires.

10. Composition selon la revendication 8 ou 9, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges.

11. Composition selon l'une quelconque des revendications 8 à 10, **caractérisée en ce que** les inhibiteurs de polymérisation sont présents en des quantités allant de 10 ppm à 20%, de préférence allant de 10 ppm à 5%, et plus préférentiellement entre 10 ppm et 1% en poids du poids total de la composition.

12. Composition selon l'une quelconque des revendications précédentes, **caractérisée en ce que** le polymère en dispersion est filmifiable.

13. Composition selon l'une quelconque des revendications précédentes, **caractérisée en ce que** le polymère en dispersion est choisi parmi les polymères radicalaires, les polycondensats, les polymère d'origine naturelle et leurs mélanges.

14. Composition selon l'une quelconque des revendications précédentes, **caractérisée en ce que** le polymère en dispersion est choisi parmi les polyuréthanes, les polyuréthanes acryliques, les polyurées, les polyurée-polyuréthanes, les polyester-polyuréthanes, les polyéther-polyuréthanes, les polyesters, les polyesters amides, les polyesters à chaîne grasse, les alkydes, les polymères ou copolypolymères acryliques et/ou vinyliques, les copolymères acryliques-silicone, les polyacrylamides, les polymères siliconés, les polymères fluorés et leurs mélanges.

15. Composition selon l'une quelconque des revendications précédentes, **caractérisé en ce que** la phase grasse liquide est constituée d'huiles d'origine minérale, animale, végétale ou synthétique, carbonées, hydrocarbonées, fluorées et/ou siliconées, seules ou en mélange.

16. Composition selon l'une quelconque des revendications précédentes, **caractérisée en ce que** la phase grasse liquide est choisie parmi l'huile de paraffine ou de vaseline, l'huile de vison, de tortue, de soja, le perhydrosqualène, l'huile d'amande douce, de calophyllum, de palme, de parléam, de pépins de raisin, de sésame, de maïs, de colza,

de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras tels que le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'alcool linoléique ou linolénique, l'alcool isostéarique ou l'octyl dodécanol ; les huiles siliconées telles que les PDMS éventuellement phénylés tels que les phényltriméthicones ou éventuellement substitués par des groupements aliphatiques et/ou aromatiques, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées ; les huiles volatiles telles que l'octaméthylcyclotétrasiloxane, le décaméthylcyclopentasiloxane, l'hexadéméthylcyclohexasiloxane, l'heptaméthylhexyltrisiloxane, l'heptaméthyloctyltrisiloxane ou les isoparaffines en C8-C16, et l'isododécane.

**17.** Composition selon l'une quelconque des revendications précédentes, **caractérisée en ce que** la phase grasse contient au moins une huile volatile à température ambiante et pression atmosphérique.

**18.** Composition selon l'une quelconque des revendications précédentes, **caractérisée en ce que** l'agent stabilisant est choisi parmi les polymères séquencés, les polymères greffés, les polymères statistiques et leurs mélanges.

**19.** Composition selon la revendication 18, **caractérisée en ce que** l'agent stabilisant est choisi parmi les polymères siliconés greffés avec une chaîne hydrocarbonée ; les polymères hydrocarbonés greffés avec une chaîne siliconée ; les copolymères greffés ayant un squelette insoluble de type polyacrylique avec des greffons solubles de type acide polyhydroxystéarique ; les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins d'un polyéther ; les copolymères d'acrylates ou de méthacrylates d'alcools en C1-C4, ou d'acrylates ou de méthacrylates d'alcools en C8-C30 ; les copolymères blocs greffés
ou séquencés comprenant au moins un bloc résultant de la polymérisation d'au moins un monomère éthylénique, à une ou plusieurs liaisons éthyléniques, éventuellement conjuguées, et au moins un bloc d'un polymère vinylique ; les copolymères blocs greffés
ou séquencés comprenant au moins un bloc résultant de la polymérisation d'au moins un monomère éthylénique et au moins un bloc d'un polymère acrylique ; les copolymères blocs greffés ou séquencés comprenant au moins un bloc résultant de la polymérisation d'au moins un monomère éthylénique et au moins un bloc d'un polyéther.

**20.** Composition selon la revendication 18, **caractérisée en ce que** l'agent stabilisant est un polymère bloc greffé ou séquencé, comprenant au moins un bloc résultant de la polymérisation de diène et au moins un bloc d'un polymère vinylique.

**21.** Composition selon l'une quelconque des revendications précédentes, **caractérisée en ce que** les particules de polymère stabilisées sont présentes dans la composition à des teneurs comprises entre 0,0001 et 30% en poids, de préférence entre 0,001 et 20%, et encore de préférence entre 0,01 et 10% en poids du poids total de la composition.

**22.** Composition selon l'une quelconque des revendications précédentes, **caractérisée en ce que** la composition comprend en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, cationiques, non ioniques ou amphotères, les polymères fixants ou non, les polyols, les protéines, les vitamines, les colorants directs ou d'oxydation, les agents nacrants, les épaississants minéraux ou organiques.

**23.** Composition selon la revendication 22, **caractérisée en ce que** l'agent est encapsulé.

**24.** Composition selon l'une quelconque des revendications précédentes, **caractérisée en ce que** la composition est sous forme de lotion, de spray ou de mousse.

**25.** Utilisation d'une composition selon l'une des revendications 1 à 24 pour le traitement cosmétique des matières kératiniques.

**26.** Utilisation selon la revendication 25 pour le traitement cosmétique des fibres kératiniques telles que les cheveux.

**27.** Utilisation d'une composition selon l'une des revendications 1 à 24 pour conférer du volume aux cheveux.

**28.** Procédé de traitement des matières kératiniques, et notamment des fibres kératiniques telles que les cheveux, **caractérisé en ce qu'**il comprend une étape d'application sur les matières kératiniques de particules de polymère dispersées dans une phase grasse liquide et stabilisées en surface par un agent stabilisant, et une étape d'application sur les matières kératiniques d'au moins un monomère électrophile.

**29.** Procédé selon la revendication 28, **caractérisé en ce que** l'application des particules de polymère est réalisée avant ou après l'application du ou des monomères électrophiles.

**30.** Procédé de traitement des matières kératiniques, **caractérisé en ce qu'**on applique sur les matières kératiniques une composition utilisée dans l'une quelconque des revendications 1 à 24, en présence d'un agent nucléophile.

**31.** Procédé selon la revendication 30, **caractérisé en ce que** l'agent nucléophile est choisi parmi les composés moléculaires, les oligomères, les dendrimères ou polymères possédant des fonctions nucléophiles choisies parmi: $R_2N^-$, $NH_2^-$, $Ph_3C^-$, $R_3C^-$, $PhNH^-$, pyridine, $ArS^-$, $R-C{\equiv}C^-$, $RS^-$, $SH^-$, $RO^-$, $R_2NH$, $ArO^-$, $N_3$, $OH^-$, $ArNH_2$, $NH_3$, $I^-$, $Br^-$, $Cl^-$, $RCOO^-$, $SCN^-$, ROH, RSH, $NCO^-$, $CN^-$, $NO_3^-$, $ClO_4^-$ et $H_2O$, Ph représentant le groupe phényle, Ar représentant un groupe aryle et R représentant un groupe aryle en $C_1$-$C_{10}$.

**32.** Procédé selon la revendication 30, **caractérisé en ce que** l'agent nucléophile est l'eau.

**33.** Procédé selon l'une quelconque des revendications 30 à 32, **caractérisé en ce que** l'on applique la composition sur les matières kératiniques préalablement humidifiées à l'aide d'une solution aqueuse dont le pH a été ajusté à l'aide d'une base, d'un acide ou d'un mélange acide/base.

**34.** Procédé selon l'une quelconque des revendications 30 à 32, **caractérisé en ce que** les matières kératiniques sont pré-imprégnées à l'aide d'un agent nucléophile autre que l'eau.

**35.** Procédé selon l'une quelconque des revendications 30 à 34, **caractérisé en ce que** les matières kératiniques sont préalablement réduites avant application de la composition.

**36.** Procédé selon la revendication 35, **caractérisé en ce que** l'agent réducteur est choisi parmi le thiosulfate de sodium anhydre, le métabisulfite de sodium en poudre, la thiourée, le sulfite d'ammonium, l'acide thioglycolique, l'acide tiolactique, le thiolactate d'ammonium, le mono-thioglycolate de glycérol, le thioglycolate d'ammonium, le thioglycérol, l'acide 2,5-dihydroxybenzoique, le di-thioglycolate de diammonium, le thioglycolate de strontium, le thioglycolate de calcium, le formo-sulfoxylate de zinc, le thioglycolate d'isooctyle, la di-cystéine, le thioglycolate de monoéthanolamine.

**37.** Procédé selon l'une des revendications 30 à 36, **caractérisé en ce que** la composition comprend en outre un polymère choisi parmi le poly(méthacrylate de méthyle) et les copolymères à base de cyanoacrylates.

**38.** Procédé selon l'une des revendications 30 à 37, **caractérisé en ce que** l'application de la composition est suivie d'un rinçage.

**39.** Kit comprenant une première composition contenant au moins un monomère électrophile et éventuellement au moins un inhibiteur de polymérisation anionique et/ou radicalaire, ainsi qu'une deuxième composition comprenant une phase grasse liquide et des particules de polymère dispersées dans la phase grasse liquide et stabilisées en surface par un agent stabilisant.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2748050 A, Shearer
- US 3527224 A
- US 3591767 A
- US 3667472 A
- US 3995641 A
- US 4035334 A
- US 4650826 A
- FR 2710646 A
- EP 749747 A
- US 6224622 B
- EP 749746 A


### Littérature non-brevet citée dans la description


- Advanced Organic Chemistry. 151-161
- Advanced Organic Chemistry. 141
- *PR Wells Prog. Phys. Org. Chem.,* 1968, vol. 6, 111
- **SAYYAH.** *J. Polymer Research,* 2000, 97
- **HOPFF.** *Makromoleculare Chemie,* 1961, 95
- **DE KEYSER.** *J. Pharm. Sci,* 1991, 67
- **KLEMARCZYK.** *Polymer,* 1998, 173
- **BRETON.** *Biomaterials,* 1998, 271
- **COUVREUR.** *Pharmaceutical Research,* 1994, 1270
- **BACHRACH.** *European Polymer Journal,* 1976, 563
- **GIPSTEIN.** *J.Org.Chem,* 1980, 1486
- **BOOR.** *J.Polymer Science,* 1971, 249
- **KANGA.** *Polymer preprints (ACS, Divison of Polymer Chemistry,* 1987, 322
- **BONNER.** *Polymer Bulletin,* 1992, 517
- **KOBAYASHI.** *Journal of Polymer Science, Part A: Polymer Chemistry,* 2005, 2754
- **WANATABE.** *J.Polymer Science : Part A :Polymer chemistry,* 1994, 2073
- Solubility parameter values. **ERIC A. GRULKE.** Polymer Handbook. 519-559
- **C. M. HANSEN.** The three dimensional solubility parameters. *J. Paint Technol.,* 1967, vol. 39, 105